# EUROPEAN PATENT APPLICATION

(11) **EP 1 300 684 A1**
(43) Date of publication of application: **09.04.2003**
(21) Application number: 01308563.4
(22) Date of filing: 08.10.2001
(51) Int. Cl.: G01N 33/543, G01N 33/542

(54) **Homogenous ligand binding assay**

(71) Applicant: Barts and The London National Health Service Trust, Whitechapel, London E1 1BB (GB); QUEEN MARY AND WESTFIELD COLLEGE, London E1 4NS (GB)
(72) Inventor: Price, Christopher, Stapleford, Cambridge CB2 5SY (GB); Holownia, Peter, Headington, Oxford OX3 7JT (GB)
(74) Representative: Raynor, John

(57) **Abstract**

A process for detecting an analyte which is a first component of a complementary ligand-receptor pair (for example an antigen), wherein the process employs colloidal magnetic particles which have bound to their surface the first component or a second component (for example an antibody) of the complementary ligand-receptor pair.
The process comprises:-
causing the analyte to take part in a specific binding reaction with the said second component of the complementary ligand-receptor pair, in the presence of the magnetic particles, under conditions such that the magnetic particles are caused to aggregate and thereby their apparent particle size is increased. The amount of increase in apparent particle size is dependent upon the amount of analyte present. The analyte is detected by detecting the apparent increase in particle size. At least 90 % of the colloidal magnetic particles have a particle size of from 6 to 16 nm excluding the said bound component of the complementary ligand-receptor pair.

## Description

The present invention relates to a process for detecting an analyte which is one component of a specifically interacting pair of species, for example to an immunoassay to detect an antibody or an antigen. The invention also relates to a diagnostic reagent for detecting an analyte for use in such a process.

Immunoassay is one of the most widely used analytical techniques for the measurement of substances of biological and clinical interest. Immunoassay methods are based on the specific recognition of epitope(s) on the antigen surface by the complementary sites (paratopes) of an antibody. This process may be made to occur in an antigen dose dependent fashion by manipulation of the amounts of antibody present. The methods used to probe, amplify and then quantify this interaction are the subject of a plethora of analytical techniques and strategies, all of which seek to achieve the common aims of simplicity, robustness, miniaturisation and speed of performance together with specificity, accuracy, precision and sensitivity of measurement.

Immunoassay quantifies a physical characteristic reflecting the amount of antigen analyte in an antibody-antigen interaction in which the key reagent is the antibody. Alternatively, the analytical system can be formulated for the detection and quantitation of antibody present in the sample of interest by using an antigen reagent.

Heterogeneous immunoassays require discrimination between bound and free forms of the analyte by a suitable separation step (e.g. precipitation, coupling to a solid phase, or chromatography). High specificity is achieved by an efficient wash step. However, this is difficult to automate and precision may suffer when extra analytical operations are introduced. Colloidal magnetic particles (referred to herein as "ferrofluid") may be used to label antibody reagent for a heterogeneous assay, with free and bound antigen analyte being separated by a magnetic field. Immunoassay techniques of this type are disclosed for example in US-A-5660990.

In a homogeneous assay, a signal from one of the labelled species is modulated by antibody-antigen interaction and is detected in a simple analytical procedure. Homogeneous immunoassays have the advantages of ease of automation and a rate of reaction limited only by molecular diffusion. However, the homogeneous nature of the format makes such assays prone to potential interferents.

Labelling methods have included radiochemical labelling, enzyme (and substrate or cofactor) labelling, fluorophore labelling (to enable fluorescence spectroscopy and variants such as polarisation, time resolved fluorescence, chemiluminescence), particulate labelling, redox labels, metal ions and others. Detection can be by spectrophotometry (incorporating turbidimetry), fluorescence luminescence and nephelometry, photon correlation, isotopic decay, photothermal detection, electrochemical detection, surface plasmon resonance and a few other specialised cases.

A commonly used detection system for homogeneous immunoassays is particle-enhanced turbidimetric immunoassay (PETIA). This is based on the particulate enhancement of light scattering during immunoagglutination. Immunoagglutination is the process whereby a protein antigen, which can be considered as multivalent, frequently with multiple copies of the same epitope as well as different and distinct epitopes, can produce a large immune complex comprising several antigen and antibody molecules. Where the antibody or antigen molecules are attached to particles, immunoagglutination will result in a large change in size of the species present, which may be detected by light scattering, for example in turbidimetry. Labelling with colloidal particles is a preferred option, since the large surface area to volume ratio provided by small particles serves to maximise speed and sensitivity.

The most common label employed in turbidimetric and nephelometric assays is the latex particle. A wide range of particles have been reported with different monomers employed, the use of a core/shell approach, different average particle diameters, and different surface residues on the particles. In the case of antibody particles that are labelled, both adsorption and covalent coupling approaches have been employed. The key limitation of latex particles is the size of the particles, with the lowest diameter in commercial use being 38 nm (before coupling of protein), with particles ranging up to in excess of 1000 nm. The diameter of the particle determines the surface area available for coupling with protein. In addition, larger particles tend to sediment, requiring reagents to be agitated frequently to maintain a homogeneous distribution of particles. A description of the use of latex particles in immunoassays is given in C. P. Price and D. J. Newman, Chapter 18, Light Scattering Immunoassay in Principles and Practice of Immunoassay, Macmillan, 2^{nd} Edition, 1997, p. 466.

The surface characteristics of the particle and means of coupling to the antibody or antigen are critically important to assay performance. Adsorbed protein may leach gradually, resulting in a gradual change in analytical performance, whereas covalent coupling can give prolonged reagent stability. The nature of the bare particle surface can influence the predisposition of the particle to non-specific aggregation in the presence of higher sample concentrations of proteins, e.g. rheumatoid factor and immunoglobulins. Metal and carbon sols, particularly gold sols, have been employed as light scattering immunoassay labels. Gold sol particles are typically between 5-100 nm in diameter. The majority of successful applications have been with analytes in urine; non-specific aggregation causes major problems in undiluted serum samples because of the much higher protein concentration. Furthermore, proteins are attached to gold sol particles by adsorption, with the possibility of problems associated with slow leaching. The absence of reactive residues on the gold particle surface means that covalent coupling is not possible, and the inability to bind haptens to particles precludes the use in competitive immunoassays for small analytes. The use of gold sol particles in immunoassays is discussed in the C. P. Price reference above.

H. H. Weetall and A. K. Gaigalas, *Anal. Lett.,* **1992,** *25*, 1039-1052, discloses the use of BSA-coated ferrofluid particles in an immunoassay. However, even before coating the particles have an average diameter of 40-60 nm. The polymer coating further increases the diameter, leading to the disadvantages of large size which apply to latex particles.

The present invention is directed towards an assay process wherein the reagent particles have the small size of the lower range of gold sol particles combined with the possibility of covalent binding provided by latex particles. It has been discovered that ferrofluids (colloidal suspensions of magnetic iron oxide nanoparticles, typically 2-20 nm in number average particle size) can provide a covalently bound light scattering label for homogeneous immunoassay based on immunoagglutination, replacing latex or gold as an enhancer of light scatter. Significantly increased sensitivity and rates of immunoagglutination are demonstrated when ferrofluid nanoparticles are used.

In its first aspect, the present invention provides a process for detecting an analyte which is a first component of a complementary ligand-receptor pair comprising the said first component and a second component capable of taking part in a specific binding reaction with the said first component, wherein the process employs colloidal magnetic particles which have a said first or second component of the complementary ligand-receptor pair bound to their surface by a linkage including a covalent bond, comprising the steps of: causing the analyte to take part in a specific binding reaction with the said second component of the complementary ligand-receptor pair, wherein the said specific binding reaction takes place in the presence of the magnetic particles, under conditions such that the magnetic particles are caused to aggregate and thereby their apparent particle size is increased, the amount of increase in apparent particle size being dependent upon the amount of analyte present, and detecting the analyte by detecting the apparent increase in particle size, wherein at least 90 % of the colloidal magnetic particles have a particle size of from 6 to 16 nm excluding the said bound component of the complementary ligand-receptor pair.

The second component of the complementary ligand-receptor pair may be bound to the surface of the colloidal magnetic particles, such that the apparent particle size increases with analyte concentration (direct assay).

Alternatively, the first component of the complementary ligand-receptor pair may be bound to the surface of the colloidal magnetic particles, such that specific binding of the second component of the complementary ligand-receptor pair with the first component bound to the said magnetic particles causes aggregation of the said particles, and wherein the presence of free analyte competes in the said specific binding reaction with the said bound first component, whereby the apparent increase in particle size decreases with increasing analyte concentration (competitive assay).

The terms "ligand" and "receptor" are used herein to denote a complementary pair of substances which are capable of recognising the spatial and charge configuration of one another, resulting in specific binding with one another. For example, where the analyte is a ligand it may be an antigen, in which case the receptor may be a complementary antibody. An example of a suitable antigen-antibody pair is human C-reactive protein (CRP) and an IgG class immunoglobulin selected for its immunological response against the antigen CRP present in the human circulation.

Where the analyte is a receptor it may be an antibody, in which case the ligand may be a complementary antigen.

The analyte ligand may also be a DNA or RNA strand, in which case the receptor may be a DNA or RNA strand containing a complementary sequence.

"Particle size" is defined as the maximum dimension of the colloidal magnetic particles as determined for dry particles by physical measurement of the maximum particle dimension on an electron micrograph. Particle size determined in this way is likely to differ from particle size measured on particles in suspension, for example using a zeta sizer. Zeta sizers do not give accurate size measurements for particles less than 40 nm in diameter.

The apparent size of the magnetic particles is preferably detected by measuring the light scattering of the sample using a spectrophotometer by turbidimetry or nephelometry, or by optical sensors using surface plasmon resonance, or by acoustic sensors using surface acoustic wave modulation, or by dynamic light scattering and electrophoretic mobility using photocorrelation spectroscopy, or by a magnetic biosensor using superconducting quantum interference, or by light intensity fluctuation in a magnetic field using reflectance polarisation, or by a decrease in light scattering at defined angles using particle counting techniques.

The magnetic particles are preferably magnetite particles.

The covalent bond by which the said first or second component of the complementary ligand-receptor pair is covalently bound to the colloidal magnetic particles is preferably formed between the said component and a coordinating species, wherein the said coordinating species is coordinated to the colloidal magnetic particles.

The coordinating species is preferably derived from citrate. The coordinating species may alternatively be derived from succinate, glutarate, tartrate, azalate, EDTA or EGTA, or hydroxyl. The coordinated groups are preferably attached to the ligand or receptor *via* an amide linkage (or an ester linkage in the case of hydroxyl) formed by a carbodiimide coupling reaction.

The step of causing the analyte to bind specifically to the ligand or receptor may optionally be accelerated by carrying the step out in the presence of polyethylene glycol, and/or carrying the step out in the presence of one or more of an alternating electric field, an alternating magnetic field and an ultrasonic wave field.

In its second aspect, the present invention relates to a process for detecting an analyte which is a first component of a complementary ligand-receptor pair comprising the said first component and a second component capable of taking part in a specific binding reaction with the said first component, wherein the process employs colloidal magnetic particles which have the said second component of the complementary ligand-receptor pair bound to their surface by a linkage including a covalent bond, comprising the steps of: causing the analyte to take part in a specific binding reaction with the said bound second component of the complementary ligand-receptor pair, under conditions such that the magnetic particles are caused to aggregate and thereby their apparent particle size is increased, the amount of increase in apparent particle size being dependent upon the amount of analyte present, and detecting the analyte by detecting the increase in apparent particle size, wherein at least 90 % of the colloidal magnetic particles have a particle size of from 6 to 16 nm excluding the said bound component of the complementary ligand-receptor pair.

In its third aspect, the present invention relates to a process for detecting an analyte which is a first component of a complementary ligand-receptor pair comprising the said first component and a second component capable of taking part in a specific binding reaction with the said first component, using colloidal magnetic particles which have the said first component of the complementary ligand-receptor pair bound to their surface by a linkage including a covalent bond, comprising the steps of:
causing the analyte to take part in a specific binding reaction with the said second component of the complementary ligand-receptor pair in the presence of the magnetic particles, such that the presence of free analyte competes in the said specific binding reaction with the said bound first component, and such that specific binding of the second component of the complementary ligand-receptor pair with the first component bound to the said magnetic particles causes aggregation of the said particles and thereby their apparent particle size is increased, whereby the apparent increase in particle size decreases with increasing analyte concentration, and detecting the analyte by detecting the apparent particle size, wherein at least 90 % of the magnetic particles have a particle size of from 6 to 16 nm excluding the said bound component of the complementary ligand-receptor pair.

In its fourth aspect, the present invention relates to a process for detecting an analyte which is a first component of a complementary ligand-receptor pair comprising the said first component and a second component capable of taking part in a specific binding reaction with the said first component, wherein the process employs colloidal magnetic particles which have a said first or second component of the complementary ligand-receptor pair bound to their surface by a linkage including a covalent bond, comprising the steps of: causing the analyte to take part in a specific binding reaction with the said second component of the complementary ligand-receptor pair, wherein the said specific binding reaction takes place in the presence of the magnetic particles, under conditions such that the magnetic particles are caused to aggregate and thereby their apparent particle size is increased, the amount of increase in apparent particle size being dependent upon the amount of analyte present, and detecting the analyte by detecting the apparent increase in particle size, wherein at least 90 % of the colloidal magnetic particles have a particle size of from 16 to 30 nm including the said bound component of the complementary ligand-receptor pair.

Preferably, the analyte is an antigen ligand, and the complementary antibody receptor is bound to the colloidal magnetic particles.

In its fifth aspect, the present invention relates to a diagnostic reagent for detecting an analyte which is a first component of a complementary ligand-receptor pair comprising the said first component and a second component capable of taking part in a specific binding reaction with the said first component by a direct assay process as described herein, comprising magnetic particles at least 90 % of which have a particle size of from 6 to 16 nm excluding the said bound component of the complementary ligand-receptor pair bound to the second component of the complementary ligand-receptor pair by a linkage including a covalent bond.

In its sixth aspect, the present invention relates to a diagnostic reagent for detecting an analyte which is a first component of a complementary ligand-receptor pair comprising the said first component and a second component capable of taking part in a specific binding reaction with the said first component by a competitive assay process as described herein, comprising magnetic particles at least 90 % of which have a particle size of from 6 to 16 nm excluding the said bound component of the complementary ligand-receptor pair bound to the first component of the complementary ligand-receptor pair by a linkage including a covalent bond.

The diagnostic reagent preferably further comprises unbound second component of the complementary ligand-receptor pair.

Preferably, the magnetic particles are suspended in buffer solution.

In its seventh aspect, the present invention relates to a method of preparing a diagnostic reagent for a ligand or receptor analyte, comprising the steps of reacting magnetic particles at least 90 % of which have a particle size of from 6 to 16 nm excluding the said bound component of the complementary ligand-receptor pair with a bifunctional coordinating species, and coupling the species to the first or second component of the complementary ligand-receptor pair using a carbodiimide reagent.

In its eighth aspect, the present invention relates to a diagnostic reagent for detecting an analyte which is a first component of a complementary ligand-receptor pair comprising the said first component and a second component capable of taking part in a specific binding reaction with the said first component by a direct assay process as described herein, comprising magnetic particles at least 90 % of which have a particle size of from 16 to 30 nm including the said bound component of the complementary ligand-receptor pair bound to the second component of the complementary ligand-receptor pair by a linkage including a covalent bond.

In its ninth aspect, the present invention relates to a diagnostic reagent for detecting an analyte which is a first component of a complementary ligand-receptor pair comprising the said first component and a second component capable of taking part in a specific binding reaction with the said first component by a competitive assay process as described herein, comprising magnetic particles at least 90 % of which have a particle size of from 16 to 30 nm including the said bound component of the complementary ligand-receptor pair bound to the first component of the complementary ligand-receptor pair by a linkage including a covalent bond.

A preferred embodiment of the invention will now be described with reference to the following drawings, in which:-
Figure 1 shows a citrated ferrofluid reagent surface of the preferred embodiment of the present invention.
Figure 2 shows a reaction scheme for forming a ferrofluid reagent of the preferred embodiment of the present invention.
Figure 3 shows UV/VIS spectra for (a) ferrofluid standard and (b) synthesised material used to prepare the reagent of the present invention.
Figure 4 shows FTIR analysis spectra for (a) ferrofluid standard and (b) synthesised material used to prepare the reagent of the present invention.
Figure 5 shows an electron micrograph and a size distribution graph of raw synthesised ferrofluid.
Figure 6 shows an electron micrograph and a size distribution graph of size-sorted ferrofluid.
Figure 7 shows a calibration curve for the citrated form of the particle reagent of the preferred embodiment of the present invention.

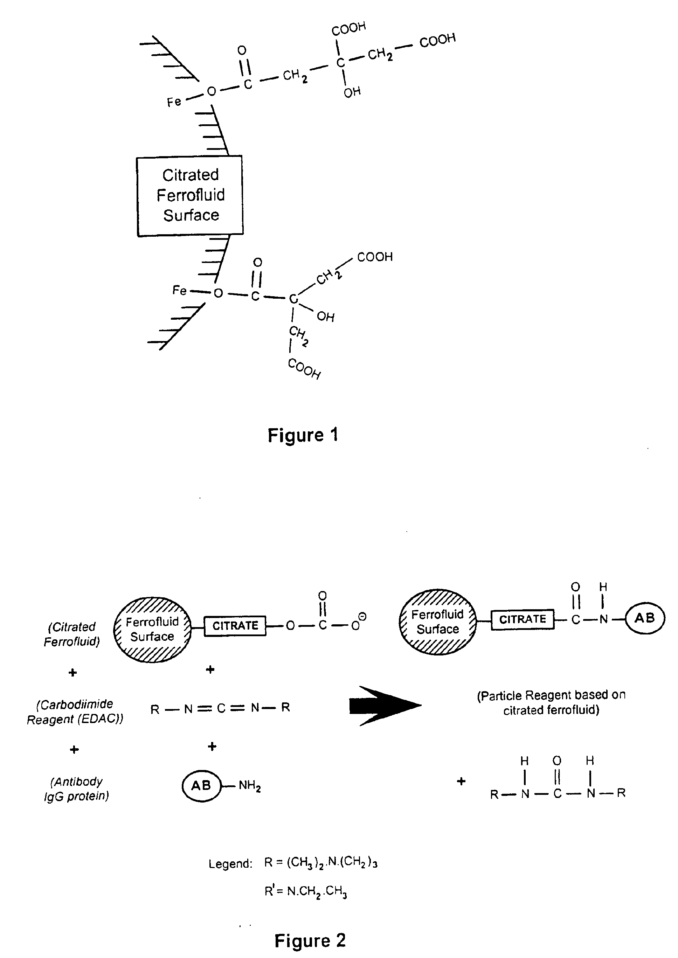

To generate the reagent used in the preferred aspect of the present invention, large biomolecules (antibodies or antigens) are directly coupled by covalent bonds to a modified surface of ferrofluid particles, comprising coordinate bound citrate ions (Figure 1). A nanosized ( 20nm), high refractive index (2.56) particle reagent is thus generated. Upon incubation with biological samples containing the immunoreactive analyte, immunospecific agglutination proceeds rapidly, and is monitored on conventional spectrophotometers by changes in turbidimetry i.e. absorbance at non-absorbing wavelengths. The term "biological samples" used in this invention refers to serum or plasma derived from clotted or anticoagulated blood respectively by centrifugal separation from the cellular fraction, or to urine.

Ferrofluid with covalently attached biomolecules provides a diagnostically effective reagent for the clinical measurement of analyte concentration. The amount of antibody per particle is typically in the order of 5-15 molecules/particle of IgG type polyclonal antibody of average affinity (10⁹ L/mol), at working assay concentrations typically of around 0.005 % w/v, sufficient for measuring analytes in the µg-mg/L range. Antibodies suitable for the invention include polyclonal antiserum for binding of antigen or hapten together with monoclonal antibodies for multi-epitopic antigens, and antibody mimics.

Nanosized particles in solution based immunoassays are preferred; for a given loading of coupled antibody, small particles provide a greater surface area to volume ratio than large particles, increasing the effective antibody concentration which, by the law of mass action, generates faster rates of reaction. Size *per se* has no influence on the rate of reaction as an increase in size may slow Brownian motion, but this is compensated for by the reduction in the distance required for diffusion. A colloidally stable 20 nm sized particle reagent of this invention is achieved by charge stabilisation in buffer systems, e.g. phosphate (pH range 5-10), of moderate ionic strength (e.g. 10-200 mM), containing between 0.001 and 5% w/v polyelectrolyte, (e.g. polyethylene glycol, PEG). This produces a zeta potential of -40 to -50 mV within a pH range 7-11 due to repulsion between the ionised carboxyl groups from unbound citrate together with acidic groups on the bound protein antibody, given that the pI of IgG antibodies is commonly around pH 6-7. At 2-3 pH units away from the point of zero charge, particles show high charge densities of 0.2 Coulombs (C) m⁻², approximately equivalent to a zeta potential of 40-50 mV in magnitude, depending on the conditions of ionic strength and species. If the potential energy barrier is high compared with the thermal energy (KT) of the particles, the suspension will be stable. In general a barrier of >25 KT, which corresponds to a zeta potential >30 mV, is sufficient to stabilise a suspension indefinitely. Even when the barrier is high enough to prevent the particles from moving into the primary minimum, they may still be trapped in the secondary minimum (i.e. flocculation) if this is deep compared with the thermal energy of the particles. However, as the secondary minimum is shallow for iron oxides (<KT) the flocculation, if allowed to occur, is readily reversible by agitation. The advantage of using citrated ferrofluid is that the charge will be at a maximum in the pH range 6-12, (falling comfortably within the physiological pH range), as well as being greater in magnitude due to the presence of two available carboxyl groups instead of one hydroxyl.

The size of the charge stabilised ferrofluid particles of the preferred embodiment of the present invention contrasts with the ferrofluids currently used in biomedical applications such as cell sorting, contrast agents for tissue imaging or heterogeneous immunoassay, where colloidal stability is achieved *via* steric stabilisation by coating the ferrofluid surface with detergent or a polymer shell. This process generates much larger reagent particles. After coupling of biomolecules onto the shell via appropriate functional groups, the current particle reagents typically have a size in the range of 150-3000 nm. An exception to this is the particles used as contrast agents for histochemical detection described in US-5075100-A, where IgG protein is coupled to ferrofluid particles via coordinated cacodylic acid, giving a particle diameter of 3-5 nm.

Light scattering intensity and distribution for colloids are governed by Rayleigh, Rayleigh-Debye and Mie laws of physics, depending on particle size, wavelength, polarisation plane, difference in refractive index from medium, concentration, angle and shape. For a given detection system such as turbidimetry or nephelometry, optimisation of these variables during immuno- agglutination is necessary for any potential immunoassay. Turbidimetry measures the change in the light transmission in the plane of incident light, (effectively an absorbance change), whilst nephelometry measures the portion of light scattered at defined angles from the incident plane (e.g. 90°). Turbidimetric assays using unpolarised light are preferred in clinical analysis because of the wide availability of rapid, automated and high precision spectrophotometers based on absorbance measurement in the UV/VIS region; there is also minimal interference from a variable background matrix found for example in serum. A change in particle size occurs during immunoagglutination; for particles <1/10th the size of the wavelength, the intensity of scatter is inversely related to the 4th power of the wavelength (Rayleigh scatter), directly proportional to the refractive index and the scattering distribution of intensity forms a symmetrical dumbbell shape. With larger initial sizes relative to the wavelength, light is scattered in a forward direction because of an increase in diffraction with size increase (Rayleigh-Debye scatter). Mie scattering, which becomes independent of wavelength, is observed at sufficiently large sizes. To obtain maximum sensitivity in signal, small particles at the shorter wavelengths and high refractive index are desirable. This is in addition to the advantage of increased reaction rate achieved with small particles as described earlier. Previous work with latex particles has indicated that there is at least tetramer formation at the end point of the immunoagglutination, therefore wavelengths of 320-340 nm at particle sizes 10-30 nm would produce the greatest signal differences during aggregation, given the practical limitation of light absorbtion of biological compounds at wavelengths <300 nm. It should also be noted that high sensitivity assays will develop a greater signal change in a given time interval, allowing the time of measurement to be reduced, and more rapid assays to be developed.

Ferrofluid iron oxide core sizes are below those of the magnetic domains necessary for a material to act as a permanent magnet. Consequently, ferrofluids only exhibit magnetism when in a magnetic field, and when the field is removed the magnetism disappears virtually instantaneously. In contrast to the large sterically stabilised ferrofluid colloids described above, charge stabilised colloid particles are the size of the much smaller iron oxide core. The charge-stabilised ferrofluid particles behave thermodynamically like solute molecules, hence they diffuse and interact with solvent or other molecules in a manner analogous to macromolecules. Since each particle possesses a giant magnetic moment compared to other paramagnetic particles, such a colloid is described as "superparamagnetic".

The property of the dipoles which results in a response to magnetic fields results equally in a response to applied electric fields and a high polarizability which confers a relatively high index of refraction when interacting with the electric vector of light (nearly twice that of latex across the UV-VIS range). Another important consequence of the interaction with electric and magnetic fields is the effect of alternating current or alternating magnetic field on ferrofluids, which enhances collision rates and can therefore be used to enhance rates of immunoreaction of ferrofluid reagents.

The iron oxides magnetite (Fe₃O₄) or maghemite (Fe₂O₃) both demonstrate these characteristics. These compounds occur naturally in rocks and soils, but may be synthesised in the laboratory by coprecipitation of Fe²⁺ and Fe³⁺ chlorides under alkaline conditions.

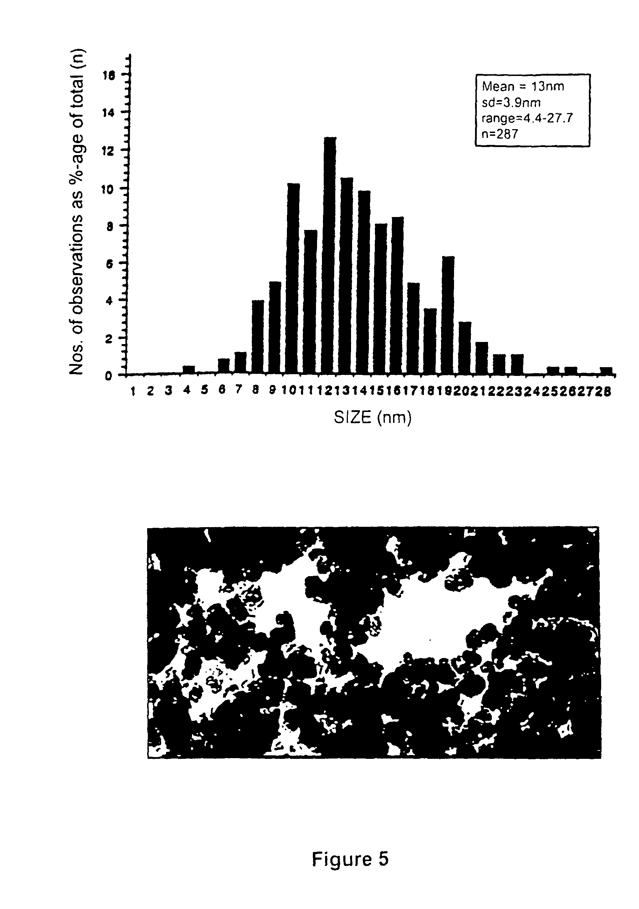

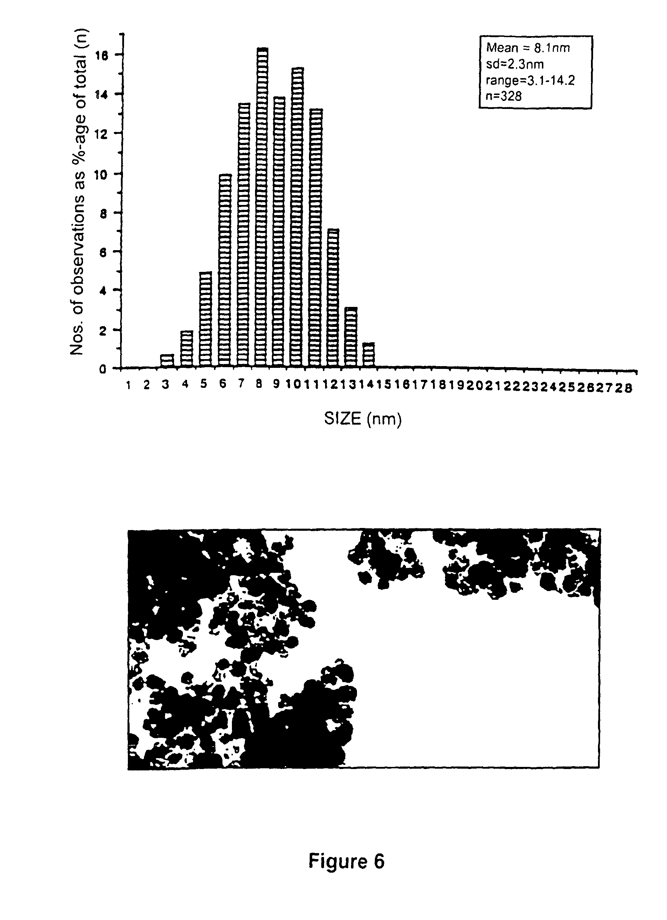

A process for precise control over size post synthesis has been developed, based on the retention of the suspended particulate phase in water after 1.5 hours. This yielded a 20% recovery from the original concentration of synthesised particles of 80 mg/ml with a size range of 8±2 nm (standard deviation) determined by electron microscopy (Figures 5 and 6). The precipitated fraction containing particles in the size range 10-25 nm is discarded.

In aqueous environments, the iron oxides contain unoccupied atomic orbitals which may participate in a coordinate bond at the surface with hydroxyl ions from water. In this invention, a coordinate bond is formed at the surface with citrate carboxyl groups, which share a lone pair of electrons from the oxygen with iron. The stability of the coordinate bonds is such that, in the case of the hydroxyl surface groups, temperatures of >250 °C for several hours are required for their removal.

The carbodiimide reaction is used to couple proteins such as antibody or antigen covalently to the surface carboxyl via amino groups, forming an amide linkage in the case of citrate ligands or an ester linkage in the case of hydroxyl ligands (Figure 2). Carbodiimide coupling of proteins to hydroxylated ferrofluid is reported in Mehta *et al., Blotech. Tech.,* **1997,** *11,* 493-496. A suitable reagent for coupling is the water-soluble condensing agent EDC at pH 5. Coupling was achieved in a range of buffered environments including NaCl, MES, PBS, HCl in the 20-100mM range. Concentrations of 2mg/ml ferrofluid, 5g/L polyclonal IgG antibody protein (raised against human C-reactive protein, CRP), and 5mg/ml EDC in buffer were observed to produce a stable particle reagent, which at a coupling efficiency of 87% gave in the final preparation an immunologically active material sufficient to produce immunoagglutination over the clinical range of interest (0-180 mg/L).

It was found that pre-activating the ferrofluid with carbodiimide for 2 hours at room temperature before overnight incubation with antibody gave higher amounts of immunoagglutination as a result of reduced cross-linking compared with the case when all the components are present together. After incubation, four sequential centrifugal separations and wash steps under optimised conditions of suspending medium are necessary to remove unwanted reaction products and to generate a colloidally stable particle reagent which, upon the introduction of antigen, will maximise the different stages of the specific antibody-antigen interaction leading to immunoagglutination. It should be recognised that the environmental conditions will vary depending on the antibody. For example, isoelectric points for antibodies may vary, hence the need for re-optimisation if the source of the antibody is changed. The use of an optimal polyethylene glycol 6000 at 0.001% w/v is particularly desirable. PEG promotes long-term particle reagent stability by increasing the buoyancy of particles in suspension as well as acting as a rate and precipitant accelerator during immunoagglutination by a mechanism of steric exclusion. Other polymers such as mannitol, glycerol and dextrans can also be used to enhance homogeneity of particle reagents.

The described conditions were found to be applicable to both citrated and hydroxylated forms of ferrofluid.

Either a direct or an inhibitory immunoassay can be performed, depending on the antigenic nature of the analyte. For example, in a direct test, when an anti-CRP antibody labelled ferrofluid is mixed with a serum sample obtained from patient blood, the rate of immunoagglutination measured will be directly related to the CRP concentration and can therefore be used as a diagnostic test of infection, inflammation or risk stratification with respect to cardiovascular disease.

An indirect test may be performed when the analyte, for example the hapten theophylline, is directly coupled to the carrier ferrofluid and can be used to monitor its circulating levels following administration. In this case the addition of an anti-theophylline antibody (raised against a protein conjugated form of the drug) is required to initiate competition between native and ferrofluid bound forms of the analyte, where the rate of immunoagglutination will be inversely related to the concentration of theophylline. Results can be obtained within 2-3 minutes for both forms of immunoassay, the procedure is simple and easy to automate onto many instrumental platforms with spectrophotometric detection (turbidimetry) or the less common nephelometry at defined angles from the plane of incident light with or without laser light. Other optical configurations to which this invention could be adapted include filter paper, optical on a tile, electrophoretic mobility, particle counting, reflectance polarisation, surface plasmon resonance, acoustic wave modulation, photon correlation spectroscopy or superconducting quantum interference.

Methods for coupling antibodies and antigens to latex particles and measurement thereof are described in US-4703018-A. The field of immunoassay and its clinical applications is reviewed in C. P. Price *et al., Principles and Practice of Immunoassay,* Macmillan, **1995,** 299-324 and 443-480 and C. P. Price, *Clin. Chem. Lab. Med.,* **1998,** *36,* 341-347. Characteristics of ferrofluids are described in J. C. Bacri *et al*., *J. Mag.* & *Mag. Mat.,* **1990,** *85*, 27-32.

The invention is illustrated by the following examples.

### (1) Synthesis & preparation of magnetite ferrofluid.

Magnetite ferrofluid was prepared by the method of coprecipitation of ferric and ferrous ions under alkaline conditions. Using freshly made solutions, 100 ml of 1 M FeSO₄ was added to 2 M FeCl₃ and mixed continuously for 5 min at room temperature (10-30 °C). The mixture was then rapidly added to 200 ml 8 M ammonia with continuous stirring, resulting in formation of a black precipitate which was heated to 80±5 °C for 30 min and then washed three times, after magnetic separation, with 500 ml of hot distilled water. The preparation was then left overnight with an added 20 ml of 8 M ammonia (pH 10). A further three wash steps with water at room temperature, (using separation by centifugation at 5000 rpm for 10 min at room temperature and resuspension with a pasteur pipette), gave the final preparation at pH 8.9 in a 250 ml volume which was stored in the dark at room temperature. Ten 0.5 ml aliquots were withdrawn and freeze-dried overnight in pre-weighed sample boats. After removal of the aqueous fraction, the weights gave a mean concentration of ferrofluid 91±0.7 mg/ml (standard deviation).

Spectral characteristics of synthesised ferrofluid were analysed by both UV/VIS and FTIR spectroscopy and compared to known spectra both from the literature and from commercially available ferrofluid standards, for example 10nm sized ferrofluid manufactured by the more traditional milling process.

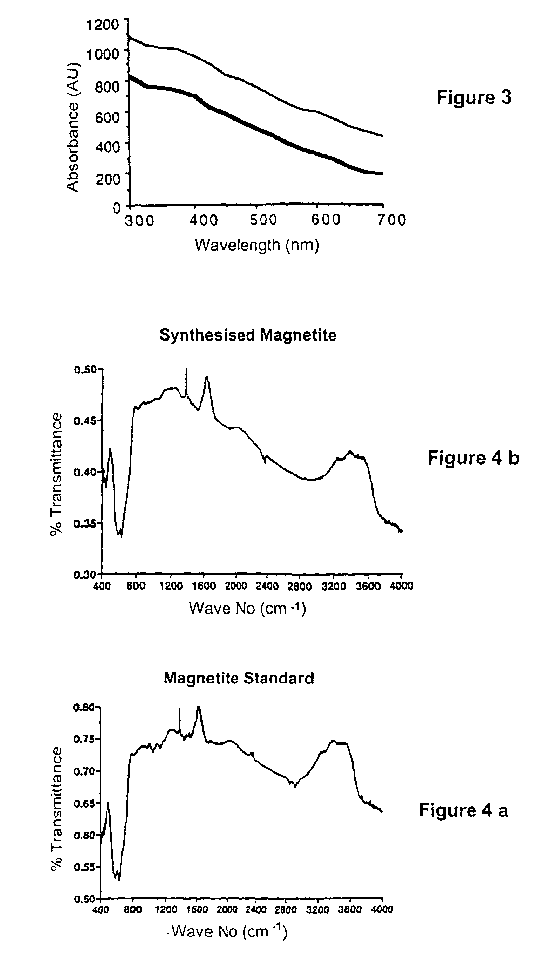

The UV/VIS spectra shown in Figure 3 were obtained using a Jasco scanning spectrophotometer between 300-700nm wavelengths for a suspension of ferrofluid in water, colloidally stable throughout the duration of the scan (5 minutes).

The FTIR analysis spectra shown in Figure 4 were obtained from dried ferrofluid samples samples ( 5mg), diluted in 1.5g of KBr crushed into powder form and analysed by a Nicolet 800-FTIR spectrophotometer used in diffuse reflectance mode (due to the poor transmittance characteristic of crystalline samples). 64 spectral scans were found to be sufficient for Fourier Transform (FT) to provide high sensitivity average spectra.

Electron micrographs of the raw synthesised ferrofluid and size-sorted ferrofluid (as prepared in Step 2) were obtained by image analysis on 0.5 µg dried ferrofluid at an EM magnification of 1:150,000, and size distribution graphs were prepared (Figures 5 and 6).

The particle size was determined by locating and then capturing an image in a pre-calibrated field of view using a JCV colour video camera (TK-1280). An Apple Mac interface card then allowed further size analysis of the image using a software programme (MIC system II, 1990-3 Videologic version 1.2.1) by manually fitting calibrated lines on screen to the expanded image of the particle diameters using the mouse control.

### (2) Surface co-ordination by citrate and other carboxylic acids, and hydroxyl.

Size sorting of particles was achieved by the method ' described earlier, which yielded a 100 ml suspension of ferrofluid at 20 mg/ml coordinated with surface hydroxyl groups. To achieve citrate surface coordination, the ferrofluid was centrifuged at 2500 rpm for 10 min, and half the supernatant was removed and replaced with a 25 mM citric acid solution. Resuspension using a 10 s ultrasound burst in a sonic bath was followed by brief agitation by shaking. The same procedure is applicable to the other carboxylic acids listed above. The mixture was left stirring gently overnight at room temperature on a rotary mixer. Excess citrate was removed by four further centrifugation/wash steps with water (20,000 rpm for 20 min, room temperature).

The final medium consisted of 50 mM phosphate buffered saline (PBS), pH 5 ready for the conjugation step.

### (3) Covalent coupling of human anti-CRP polyclonal IgG antibody to citrated ferrofluid.

Turbidimetric grade polyclonal anti-CRP (IgG fraction) was obtained from Binding Site Ltd (Cat. No. PN044). Following an overnight dialysis of 20 ml of the anti-CRP in 500 ml of 50 mM sodium phosphate buffer saline (0.9% NaCl) pH 5.0 (PBS), the dialysed antibody was spun at 2500 rpm for 5 min and the resulting supernatant removed and retained from the particulates in the pellet. The protein concentration was measured spectrophotometrically at 280 nm from 10 replicates of 50 µL aliquots of 1:50 dilutions in the buffer and was 13.8±0.17 mg/ml (standard deviation). Coupling was performed by incubating 5.85 ml PBS, 1.5 ml citrated ferrofluid (1 mg/ml) and 150 µL of 5 mg/ml 1-ethyl-3-dimethyl-carbodiimide (EDC) at 30 °C for 2 hours in an orbital shaker. The ferrofluid reagent was centrifuged at 20000 rpm for 20 min at room temperature, washed twice in PBS buffer and finally resuspended in 3 ml to which was added 1.1 ml antibody and 1.5 ml PBS containing 0.04 % Gafac, and then incubated overnight at room temperature in an orbital shaker. The mixture was then washed three times in 0.2 M glycine buffered saline with 0.9 % NaCl (GBS), containing 1 % bovine serum albumin (BSA) and 0.004 % sodium azide, pH 7.5 with the final resuspension prepared by sonicating twice for 15 s at 20 µ intensity in an ice bath with a 1 min gap between bursts. The solution was stored at 4 °C ready for use. An aliquot of supernatant from the first centrifugation was retained for measurement of protein concentration so that the amount bound to the ferrofluid particles could be calculated by subtraction from the original amount used.

Working dilutions of ferrofluid reagent in GBS pH 7.5 were obtained from the absorbance spectrum by a response of 1000 mAU (±100) at 340 nm. This corresponded to a 1:10 dilution of the ferrofluid particle reagent which was ready for use in the immunoassay. The reasons are analogous to those used in normal latex particle immunoassays; a) a turbidimetric response indicating immunoagglutination must lie within linearity of the Beer-Lambert law (0-2000 mAU range); b) a certain amount of background turbidity must be tolerated due to presence of unreacted particle reagent at the start, concentrations and surface coverage of antibody being then optimised to give a maximum response, both in terms of signal, reaction time & stability; the starting 1000 mAU absorbance thus reflecting a compromise between the two; c) Monitoring agglutination is selected at 340 nm wavelength (in the UV/VIS range) because this shows the greatest signal change according to the law of Rayleigh light scattering for <70 nm initially sized particles.

### (4) Measurement of immunoagglutination in an homogeneous immunoassay for CRP.

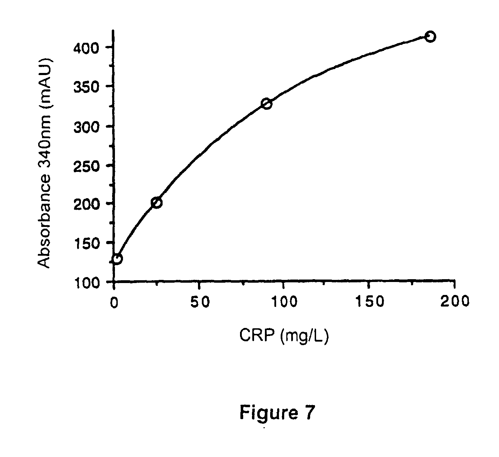

Using the ferrofluid particle reagent (PR) generated by the previous procedure, the following incubation mixture was prepared in a 2 ml cuvette: 1.6 ml PR, 200 µL PBS containing 0.2 % PEG pH 7.5. The mixture was placed inside a Jasco 7850 UV-VIS spectrophotometer capable of monitoring absorbance changes over time at a fixed wavelength of 340 nm. The reaction was started by adding a 40 µL volume of sample (serum or plasma) containing the CRP analyte, followed by rapid mixing. The progress of the immunoagglutination was monitored by the increase in absorbance (mAU) over time, where the response was calculated after a standard reaction period defined as the difference (mAU) observed for the progress curve between the plateau region at near completion and the initial point after mixing. The time interval was typically 3 min. The responses were recorded for five CRP serum calibrants reflecting the analytical range required in clinical analysis (Figure 7 represents the calibration curve).

## Claims

1. A process for detecting an analyte which is a first component of a complementary ligand-receptor pair comprising the said first component and a second component capable of taking part in a specific binding reaction with the said first component, wherein the process employs colloidal magnetic particles which have a said first or second component of the complementary ligand-receptor pair bound to their surface by a linkage including a covalent bond, comprising the steps of:
causing the analyte to take part in a specific binding reaction with the said second component of the complementary ligand-receptor pair, wherein the said specific binding reaction takes place in the presence of the magnetic particles, under conditions such that the magnetic particles are caused to aggregate and thereby their apparent particle size is increased, the amount of increase in apparent particle size being dependent upon the amount of analyte present, and
detecting the analyte by detecting the apparent increase in particle size,
wherein at least 90 % of the colloidal magnetic particles have a particle size of from 6 to 16 nm excluding the said bound component of the complementary ligand-receptor pair.

2. A process as claimed in Claim 1, wherein the second component of the complementary ligand-receptor pair is bound to the surface of the colloidal magnetic particles, such that the apparent particle size increases with analyte concentration.

3. A process as claimed in Claim 1, wherein the first component of the complementary ligand-receptor pair is bound to the surface of the colloidal magnetic particles, such that specific binding of the second component of the complementary ligand-receptor pair with the first component bound to the said magnetic particles causes aggregation of the said particles, and wherein the presence of free analyte competes in the said specific binding reaction with the said bound first component, whereby the apparent increase in particle size decreases with increasing analyte concentration.

4. A process as claimed in any one of the preceding claims, wherein the analyte is an antigen and the second component of the complementary ligand-receptor pair is an antibody.

5. A process as claimed in Claim 4, wherein the antigen analyte is human C-reactive protein (CRP) and the antibody is an IgG class immunoglobulin having an immunological response against CRP.

6. A process as claimed in any one of Claims 1 to 3, wherein the analyte is an antibody and the second component of the complementary ligand-receptor pair is an antigen.

7. A process as claimed in any one of Claims 1 to 3, wherein the analyte and second component of the complementary ligand-receptor pair are each DNA or RNA molecules.

8. A process as claimed in any one of the preceding claims, wherein the step of detection of the apparent increase in particle size is carried out:-
by turbidimetry or nephelometry,
by surface plasmon resonance,
by surface acoustic resonance modulation,
by reflectance polarisation,
by photon correlation spectroscopy,
by superconducting quantum interference,
by reflectance polarisation, or
by particle counting.

9. A process as claimed in any one of the preceding claims, wherein at least 90 % of the particles have a particle size of from 8 to 14 nm excluding the said bound component of the complementary ligand-receptor pair.

10. A process as claimed in Claim 9, wherein at least 90 % of the particles have a particle size of from 10 to 12 nm excluding the said bound component of the complementary ligand-receptor pair.

11. A process as claimed in any one of the preceding claims, wherein the colloidal magnetic particles are colloidal magnetite particles.

12. A process as claimed in any one of the preceding claims, wherein said covalent bond by which the said first or second component of the complementary ligand-receptor pair is covalently bound to the colloidal magnetic particles is formed between the said component and a coordinating species, wherein the said coordinating species is coordinated to the colloidal magnetic particles.

13. A process as claimed in Claim 12, wherein the coordinating species is derived from hydroxyl, citrate, succinate, glutarate, tartrate, azalate, EDTA or EGTA.

14. A process as claimed in any one of the preceding claims, wherein the specific binding process is carried out in the presence of polyethylene glycol.

15. A process as claimed in any one of the preceding claims, wherein the specific binding process is carried out in the presence of one or more of an alternating electric field, an alternating magnetic field, and an ultrasonic wave field.

16. A process for detecting an analyte which is a first component of a complementary ligand-receptor pair comprising the said first component and a second component capable of taking part in a specific binding reaction with the said first component, wherein the process employs colloidal magnetic particles which have the said second component of the complementary ligand-receptor pair bound to their surface by a linkage including a covalent bond, comprising the steps of:
causing the analyte to take part in a specific binding reaction with the said bound second component of the complementary ligand-receptor pair, under conditions such that the magnetic particles are caused to aggregate and thereby their apparent particle size is increased, the amount of increase in apparent particle size being dependent upon the amount of analyte present, and
detecting the analyte by detecting the increase in apparent particle size,
wherein at least 90 % of the colloidal magnetic particles have a particle size of from 6 to 16 nm excluding the said bound component of the complementary ligand-receptor pair.

17. A process for detecting an analyte which is a first component of a complementary ligand-receptor pair comprising the said first component and a second component capable of taking part in a specific binding reaction with the said first component, using colloidal magnetic particles which have the said first component of the complementary ligand-receptor pair bound to their surface by a linkage including a covalent bond, comprising the steps of:
causing the analyte to take part in a specific binding reaction with the said second component of the complementary ligand-receptor pair in the presence of the magnetic particles, such that the presence of free analyte competes in the said specific binding reaction with the said bound first component, and such that specific binding of the second component of the complementary ligand-receptor pair with the first component bound to the said magnetic particles causes aggregation of the said particles and thereby their apparent particle size is increased, whereby the apparent increase in particle size decreases with increasing analyte concentration, and
detecting the analyte by detecting the apparent particle size,
wherein at least 90 % of the magnetic particles have a particle size of from 6 to 16 nm excluding the said bound component of the complementary ligand-receptor pair.

18. A diagnostic reagent for detecting an analyte which is a first component of a complementary ligand-receptor pair comprising the said first component and a second component capable of taking part in a specific binding reaction with the said first component by a process claimed in any one of Claims 1, 2, and 4 to 16, comprising magnetic particles at least 90 % of which have a particle size of from 6 to 16 nm excluding the said bound component of the complementary ligand-receptor pair bound to the second component of the complementary ligand-receptor pair by a linkage including a covalent bond.

19. A diagnostic reagent for detecting an analyte which is a first component of a complementary ligand-receptor pair comprising the said first component and a second component capable of taking part in a specific binding reaction with the said first component by a process as claimed in any one of Claims 1, 3 to 15 and 17, comprising magnetic particles at least 90 % of which have a particle size of from 6 to 16 nm excluding the said bound component of the complementary ligand-receptor pair bound to the first component of the complementary ligand-receptor pair by a linkage including a covalent bond.

20. A diagnostic reagent as claimed in Claim 19, further comprising unbound second component of the complementary ligand-receptor pair.

21. A diagnostic reagent as claimed in any one of Claims 18 to 20, wherein the magnetic particles are suspended in buffer solution.

22. A method of preparing a diagnostic reagent for a ligand or receptor analyte as claimed in any one of Claims 18 to 21, comprising the steps of reacting magnetic particles at least 90 % of which have a particle size of from 6 to 16 nm excluding the said bound component of the complementary ligand-receptor pair with a bifunctional coordinating species, and coupling the species to the first or second component of the complementary ligand-receptor pair using a carbodiimide reagent.

23. A process for detecting an analyte which is a first component of a complementary ligand-receptor pair comprising the said first component and a second component capable of taking part in a specific binding reaction with the said first component, wherein the process employs colloidal magnetic particles which have a said first or second component of the complementary ligand-receptor pair bound to their surface by a linkage including a covalent bond, comprising the steps of:
causing the analyte to take part in a specific binding reaction with the said second component of the complementary ligand-receptor pair, wherein the said specific binding reaction takes place in the presence of the magnetic particles, under conditions such that the magnetic particles are caused to aggregate and thereby their apparent particle size is increased, the amount of increase in apparent particle size being dependent upon the amount of analyte present, and
detecting the analyte by detecting the apparent increase in particle size,
wherein at least 90 % of the colloidal magnetic particles have a particle size of from 16 to 30 nm including the said bound component of the complementary ligand-receptor pair.

24. A diagnostic reagent for detecting an analyte which is a first component of a complementary ligand-receptor pair comprising the said first component and a second component capable of taking part in a specific binding reaction with the said first component by a process claimed in Claim 23, comprising magnetic particles at least 90 % of which have a particle size of from 16 to 30 nm including the said bound component of the complementary ligand-receptor pair bound to the second component of the complementary ligand-receptor pair by a linkage including a covalent bond.

25. A diagnostic reagent for detecting an analyte which is a first component of a complementary ligand-receptor pair comprising the said first component and a second component capable of taking part in a specific binding reaction with the said first component by a process as claimed in Claim 23, comprising magnetic particles at least 90 % of which have a particle size of from 16 to 30 nm including the said bound component of the complementary ligand-receptor pair bound to the first component of the complementary ligand-receptor pair by a linkage including a covalent bond.
